Europäisches Patentamt

European Patent Office

Office européen des brevets

(19)

(11) Publication number: **0 090 093**
**B1**

(12) **EUROPEAN PATENT SPECIFICATION**

(45) Date of publication of patent specification: **08.06.88**

(51) Int. Cl.⁴: **A 61 M 1/14**

(21) Application number: **82112125.8**

(22) Date of filing: **30.12.82**

(54) **A humors processing device.**

(30) Priority: **26.03.82 JP 47293/82**

(43) Date of publication of application:
**05.10.83 Bulletin 83/40**

(45) Publication of the grant of the patent:
**08.06.88 Bulletin 88/23**

(84) Designated Contracting States:
**DE FR GB SE**

(56) References cited:
**EP-A-0 024 439**
**DE-A-2 321 188**
**DE-A-2 441 370**
**FR-A-2 455 462**
**GB-A-2 020 253**
**GB-A-2 021 418**
**US-A-4 043 501**

(73) Proprietor: **KAWASUMI LABORATORIES INC.**
**2-7-15, Minamirokugo Otaku**
**Tokyo (JP)**

(72) Inventor: **Kawano, Yasuhiro**
**No. 9-16, Ohgiya, 2-chome**
**Kamakura-shi Kanagawa-ken (JP)**

(74) Representative: **Klingseisen, Franz, Dipl.-Ing.**
**et al**
**Dr. F. Zumstein sen. Dr. E. Assmann Dr. R.**
**Koenigsberger Dr. F. Zumstein jun. Dipl.-Ing. F.**
**Klingseisen Bräuhausstrasse 4**
**D-8000 München 2 (DE)**

Courier Press, Leamington Spa, England.

## Description

The invention relates to a humor processing arrangement according to the preamble of claim 1.

As humor processing arrangements there have been artificial kidney, liver, lungs, curing abdominal dropsy processing device or plasma separator, which serve as substitutions for the organs of the human body. GB-A-2 021 418 discloses a medical set for an artificial kidney or oxygenator. Blood is passing through flow lines, one of them being connected With the arterial system, whereas the other line is connected with the venous system of the patient. Clamps are provided, which are operable by finger pressure to close a line. In such arrangements the humor, such as blood, circulates external of the human body and removes wasted materials in the humor by utilizing a combination of filtration, dializing, absorbing and the like.

For these arrangements membrane module or hemoperfusion cartridge are commonly employed. The former is made by assembling membranes of hollow fibres of from about several hundreds to about 10,000 of cellulose, such as cuprammonium rayon or viscose rayon, or synthetic polymer, such as polyvinylalcohol, polymethacrylate, polypropylene or polyacrylonitrile. The latter is made of petroleum-based activated charcoal or plum husk-based activated charcoal.

Said membrane or hemoperfusion cartridge is dealt with by an ethylene oxide gas sterilizing method, a gamma radiation sterilizing method, a formaline sterilizing method or a steam sterilizing method. However, since the two former methods carry out the sterilization in the dried condition, they will perhaps reduce functions of the membrane. Especially, with respect to the hollow fiber membrane, in case of the dried condition, the humor runs unsmoothly within the hollow fibers, so that air bubbles are removed.

Further in the ethylene oxide gas sterilization, residual toxicity after the sterilization · is a problem. The gamma radiation sterilization modifies or dissolves the membrane, the casing or other parts, and consequently the dissolved substances will elute into the humor. In the formalin sterilization the formalin per se is harmful to the human body. Therefore, the device should be washed for a long period of time. EP-A-0 024 439 further discloses sterilization of an artificial organ.

Recently a steam autoclave sterilizing method has been used frequently. This method has been employed not only in the humor processing arrangement, but also in indwelling catheters and the humor circulating line, such as a blood circulating line, which communicates a patient with the humor processing device. However, in this sterilization method the humor processing unit and the humor circulating line are sterilized individually and they should not be connected for use. If the humor processing unit of the wet condition is used at connection, the liquid leaks from an inlet or an outlet of the device and the device is polluted and the air will go thereinto.

Since the humor line should be soft and transparent, PVC resin forming agent containing plasticizer, such as dioctyl phthalate, are often used. However, if the resin that contains the plasticizer contacts electrolyte as a physiological saline solution, the plasticizer enters the electrolyte. If the sterilization is carried out under the condition of filling, the physiological saline solution, the filled solution, is discharged before us of the circulating line, and it is necessary to wash the line with new physiological saline solution and take out the air from the line. This is called priming.

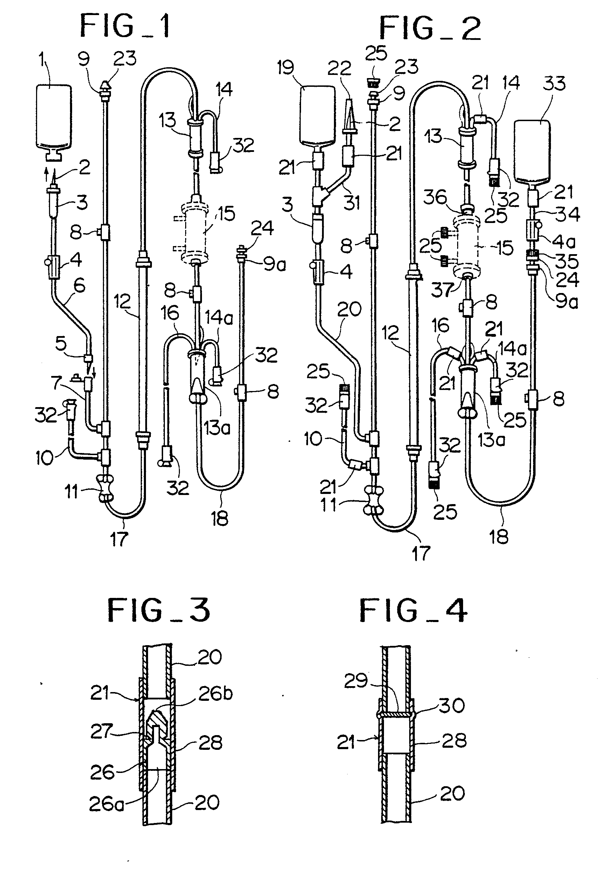

The priming is carried out, as shown in Fig. 1, by connecting an inlet circulating line 17 and an outlet circulating line 18 to the humor processing unit 15, connecting a priming set 6 having a clamp 4, a drip chamber 3 and others to a priming line 7, which branches off from the main tube of the inlet line 17, piercing an introducing needle 2 at an end point of the priming set 6 into a mouth of a bottle 1 supporting the physiological saline solution, and running the solution within the bottle into the inlet line 17, the outlet line 18 and the humor processing unit 15.

However, in such conventional methods, it is necessary to bother about disinfecting a needle piercing portion of a rubber plug in the bottle mouth, when the connector 5 is stiffly mounted at the end of the priming line 7 or the needle 2 is pierced into the mouth of the bottle. In addition a large force is required, if the introducing needle is of plastic material. Further it is necessary to cramp luer connectors 32 by means of several forceps for preventing leakage of the physiological saline solution. The prior art is troublesome in preparation requiring many processes and therefore it is unhygienic.

In addition, when the priming is started, the waste liquid is discharged from the outlet 24 of the outlet line 18 and at this time the outlet 24 is held with an adhesive tape to an edge of a vessel, such as a bucket. This manner makes the outlet 24 touch the vessel, or directly exposes it to the air. This is insanitary. Further it is troublesome to support the outlet 24 to the vessel with the adhesive tape.

It is the object of the invention to carry out steam autoclave sterilization of an arrangement according to the preamble of claim 1 under such a condition that all parts of the arrangement are connected with each other, so that the sterilization can be performed hygienically and that the costs of sterilization can be reduced.

This object is achieved by the features in the characterizing part of claim 1. With such a humor processing arrangement the preparation for priming can be performed easily and hygienically. Further it is possible to deal with the waste solution in an easy and hygienic manner.

GB-A-2 020 253 discloses a fluid flow valve, which may be opened permanently by finger pressure under sterile conditions. The flow valve is arranged in a line connected to a blood bag for opening the blood bag. No humor processing arrangement is described.

An example of the invention will be described in more detail in connection with the drawings.

Fig. 1 is an outlined view of a conventional humor processing arrangement,

Fig. 2 is an outlined view showing an arrangement according to the invention,

Fig. 3 and 4 are cross-sectional views of internal closure means according to the invention.

In Fig. 2 the numeral 15 shows a humor processing unit. The device comprises about 10,000 membranes of hollow fibers made of cellulose or synthetic polymer, and purifies the humor by filtering or dialyzing while the humor passes through the hollow fibers. The unit 15 has a humor inlet 6 and a humor outlet 37 at respective ends, which are connected to a humor inlet line 17 and a humor outlet line 18, which together form a humor line.

The lines 17, 18 are in general made of flexible synthetic resin, say PVC. A main tube of the line 17 is provided with an air trap 13, a pump tube 12 and a negative pressure monitor 11. A main tube of the line 18 is also provided with an air trap 13a. The negative pressure monitor 11 is to indicate abnormal pressure in the inlet line 17.

The air traps 13, 13a serve to remove the air in the humor and are connected to level adjustment lines 14, 14a, which keep constant levels of the humor in the air traps. The air trap 13a of the humor outlet line 18 is connected to a pressure monitor line 16, which observes the pressure of the humor under processing.

A humor inlet 23 and a humor outlet 24 are equipped with shunt adapters 9, 9a, respectively. The inlet line 17 and the outlet line 18 are provided at appropriate portions with injection sites 8 for injecting liquid medicine or sampling the humor. The inlet line 17 has a heparin line 10, which branches off from the main tube for injecting anticoagulant solution. The inlet line 17 has a branch from the main tube, a communicating tube 20 for a priming line, which is connected at its end portion with a bag 19 of soft synthetic resin filled with the physiological saline solution. The communicating tube 20 is positioned with a clamp 4, which adjusts the rate of flow of the physiological saline solution and a drip chamber 3, which allows observation of its rate of flow. From the communicating tube 20 there branches a preparatory line 31 from a point above the drip chamber 3, Which is to connect a bag or bottle supporting the physiological saline solution in case the solution runs out, which has an introducing needle 2 at its end portion with a protecting cap 22.

The bag is connected to the tube 20 via an internal closure means 21, which is easily opened by finger pressure.

Figs. 3 and 4 illustrate examples of the above-mentioned internal closure means. In Fig. 3 the tube 20 has mounted at its middle part a flexible communicating tube 28, which has a larger diameter than that of the tube 20, and the tube 28 is supported with a communicating tube 26 therein. The tube 26 is made of hard synthetic resin and has an outer diameter almost equal to an inner diameter of the communicating tube 28. With respect to the communicating tube 26, a lower end portion 26a opens and an upper end portion is closed with a head portion 26b, whose diameter is smaller than that of the tube 28, but larger than an inner diameter of the tube 20, and the head portion 26b is formed with a thin film portion 27 at its lower part. If the tube 28 is pressed by the fingers from a external side, the head is broken at the thin film 27 to communicate with the tube 20. When the head portion 26b is broken off, the broken head portion 20b does not completely close the communicating tube 20.

In Fig. 4 the communicating tube 28 is adapted under pressure with a circular communicating piece 29, which is larger than the tube 28 in diameter, so that the tube 28 holds the piece 29 by a holding portion (30), which is made by said adaption, and the piece (30) is laid by the finger pressure from the external side.

Internal closure means 21 having such constructions are also provided at the preparatory line 31 and also on the heparin line 10, the level adjusting lines 14, 14a and the pressure monitoring line 16. In a line having a protamine line, said internal closure means may be provided near a branching portion of a protaline line.

An adapter 9a of the humor outlet line 18 is connected with a waste liquid bag 33, which is made of soft synthetic resin as the bag 19. An internal closure means 21, which has the same construction as above, is positioned on a tube 34 extending to the waste liquid bag 33 and a clamp 4a is provided thereon, and a connector 35 is detachably connected with the adapter 9a.

Seals are made by means of water-tight caps or sealing members 25 on mouths of the luer connectors 32 at the ends of the branch lines 10, 14, 14a, 16, the shunt adapters 9, 9a and the humor processing unit 15, so that interior of the inlet line 17, the outlet line 18 and the processing unit 15 are completely cut off from the exterior.

Under this condition, the lines 17, 18 and the humor processing unit 15 are filled with a harmless liquid, for example, the physiological saline solution or distilled water. The solution is forced to flow thereinto by effecting pressure on the adapter 9 of the inlet line 17, or by effecting negative pressure on the outlet 24 of the outlet line 18.

The humor processing arrangement is charged with the harmless liquid, and the autoclave sterilization is carried out in saturated steam of 121°C x 20 min decided by Japanese pharmacopeia, under the condition that each of said mouths is sealed with sealing member 25 and the processing unit 15, the inlet line 17, the outlet line 18 and the waste liquid bag 22 are connected. Accordingly, raw materials for setting up the humor processing arrangement should be able to endure the temperatures of said autoclave sterilization, and be difficult to deform. For example polycarbonate is used for the outer body of the processing unit 15, polycarbonate or polypropylene are used for the sealing member 25, and PVC, silicone or polyurethane resin are used for the humor circulating lines 17, 18. For other parts, such a property of heat-resistance and anti-deformity is desirable.

A further reference will be made to the priming operation. There are, at predetermined positions, the humor lines 17, 18, the processing unit 15 and the bag 19. The sealing member 25 is taken off from the humor introducing mouth 23 of the inlet line 17, and the opened mouth is positioned at the lower place, and after the liquid in the inlet line 17 is discharged, the portion 21 at the end of the communicating tube 20 is communicated by the finger pressure to introduce the physiological saline solution from the bag 19 thereinto.

Subsequently, an internal closure means 21 near the waste liquid bag 33 of the tube 34 is opened by finger pressure, and a liquid feed pump (not shown) is operated moderately and squeezes a pump 12 thereby to force out the liquid in the inlet line 17, the processing device 15 and the outlet line 18 from the outlet 24, and while the physiological saline solution is substituted, the air introduced by the sterilization is removed.

Thus, the physiological saline solution is allowed to flow until the air is completely removed and if the prepared physiological saline solution is not enough, the introducing needle 2, would be pierced into the solution bottle or bag and the internal closure means 21 on the preparatory line 31 is broken to supplement the solution.

The waste solution is stored in the bag 33 through the humor exhausting outlet 24. After completion of the priming, the clamp 4a is closed and the connector 35 is removed from the adapter 9, and the waste solution is discharged.

The present invention has the following advantages:

1) Since it is possible to undertake the autoclave sterilization under condition of connecting all members of the processing device, humor circulating lines and bags, the connection is not required during use, and the filled solution is not leaked, and the cost of the processing sterilization is lowered.

2) It is no longer necessary to connect the priming set as conventionally and clamp it by means of forceps, and since the internal closure means is opened by finger pressure as the occasion demands, the priming preparation is made under perfect sealing conditions, and the waste solution can be dealt with easily and hygienically, and

3) It is used in an urgent case or home dialysis, which has become popular recently.

This invention may be of course applied to the humor processing units, such as the artificial kidney, liver, lungs, abdominal dropsy processing device or plasma separator.

The actual embodiments of the arrangement are not limited to those illustrated, but may be varied.

**Claims**

1. A humor processing arrangement for circulating humor external to a human body and for removing waste materials from the humor, the arrangement comprising a humor processing unit (15) for removing materials from the humor, an inlet line (17) connected with the processing unit (15), an outlet line (18) connected with the processing unit (15), and branch lines (10, 14, 16, 20) connected with the inlet and outlet lines and each having a closure means (21), one (20) of the branch lines branching off from the inlet line (17) and being connected with a bag (19) to be filled with a physiological saline solution, characterized in that the closure means (21) of the branch lines are each internal closure means (21) which prevents any leakage and after opening by finger pressure on the outside remain permanently open, and in that a waste bag (33) is connected to the end of the outlet line (18) via a further internal closure means (21) which prevents any leakage and after opening by finger pressure on the outside remains permanently open.

2. Humor processing arrangement according to claim 1, characterized in that the internal closure means (21) comprises a flexible connecting tube (28) which connects the ends of two lines by overlapping the line ends, and a communicating tube (26) inserted in the connecting tube (28) between the ends of the lines, which communicating tube (26) is closed by a head portion (26b) that can be removed from the communicating tube by finger pressure on the outside of the connecting tube (28).

3. Humor processing arrangement according to claim 1, characterized in that the internal closure means (21) comprises a circular communicating piece (29), which has a diameter larger than the inner diameter of the connecting tube (28) for closing the passage through the connecting tube (28) and which may be tilted by finger pressure on the outside of the connecting tube (28).

**Patentansprüche**

1. Vorrichtung zum Behandeln von Körperflüssigkeiten zum Zirkulieren von Körperflüssigkeit außerhalb des menschlichen Körpers und zum Entfernen von Abfallmaterialien aus der Körperflüssigkeit, wobei die Vorrichtung eine Körperflüssigkeitsbehandlungseinheit (15) zum Entfernen von Materialien aus der Körperflüssigkeit umfaßt, ferner eine mit der Behandlungseinheit (15) verbundene Eingangsleitung (17), eine mit der Behandlungseinheit (15) verbundene Ausgangsleitung (18) und Zweigleitungen (10, 14, 16, 20), die mit der Eingangsund Ausgangsleitung verbunden sind und deren jede eine Verschlußeinrichtung (21) aufweist, wobei eine (20) der Zweigleitungen von der Eingangsleitung (17) abzweigt und mit einem Sack (19) verbunden ist, der mit einer physiologischen Salzlösung zu füllen ist, dadurch gekennzeichnet, daß die Verschlußeinrichtungen (21) der Zweigleitungen jeweils interne Verschlußeinrichtungen (21) sind, die jede Undichtigkeit verhindern und die nach dem Öffnen durch Fingerdruck von außen permanent geöffnet bleiben, und daß ein Abfallsack (33) mit dem einen Ende der Ausgangsleitung (18) über eine weitere interne Verschlußeinrichtung (21) verbunden ist, die jede Undichtigkeit verhindert und nach dem Öffnen durch Fingerdruck von außen permanent geöffnet bleibt.

2. Vorrichtung zum Behandeln von Körperflüssigkeiten nach Anspruch 1, dadurch gekennzeichnet, daß die interne Verschlußeinrichtung (21) ein flexibles Verbindungsrohr (28) aufweist, welches die Enden von zwei Leitungen durch Überlappen der Leitungsenden verbindet, und ein kommunizierendes Rohr (26), welches im Verbindungsrohr (28) zwischen den Enden der Leitungen eingesetzt ist, wobei das kommunizierende Rohr (26) durch einen Kopfteil (26b) verschlossen ist, welcher von der kommunizierenden Leitung durch Fingerdruck auf die Außenseite der Verbindungsleitung (28) entfernt werden kann.

3. Vorrichung zum Behandeln von Körperflüssigkeiten nach Anspruch 1, dadurch gekennzeichnet, daß die interne Verschlußeinrichtung (21) ein scheibenförmiges Verbindungsstück (29) umfaßt, das einen größeren Durchmesser aufweist als der Innendurchmesser der Verbindungsleitung (28) zum Verschließen des Durchgangs durch die Verbindungsleitung (28) und das durch Fingerdruck auf die Außenseite der Verbindungsleitung (28) gekippt werden kann.

**Revendications**

1. Agencement de traitement de liquides du corps pour mettre en circulation les liquides du corps à l'extérieur d'un corps humain et pour évacuer les substances usées des liquides du corps, l'agencement comprenant une unité de traitement des liquides du corps (15) pour éliminer des substances des liquides du corps, une conduite d'entrée (17) reliée à l'unité de traitement (15), une conduite de sortie (18) reliée à l'unité de traitement (15), et des conduites de dérivation (10, 14, 16, 20) reliées aux conduites d'entrée et de sortie et comprenant chacune un moyen à fermeture (21), l'une (20) des conduites de dérivation partant de la conduite d'entrée (17) et étant reliée à un sac (19) à remplir avec une solution saline physiologique, caractérisé en ce que les moyens à fermeture (21) des conduites de dérivation sont chacun des moyens à fermeture internes (21), qui évitent toute fuite et après ouverture par une pression appliquée par les doigts sur le côté extérieur restent ouverts en permanence, et en ce qu'un sac à liquides usés (33) est relié à l'extrémité de la conduite de sortie (18) par l'intermédiaire d'un autre moyen à fermeture interne (21), qui évite toute fuite et, après ouverture par une pression appliquée par les doigts sur l'extérieur, reste ouvert de façon permanente.

2. Agencement de traitement des liquides du corps selon la revendication 1, caractérisé en ce que le moyen à fermeture interne (21) comprend un tube de connexion flexible (28) qui relie les extrémités de deux conduites en chevauchant les extrémités des conduites, et un tube de communication (26) intégré dans le tube de connexion (28) entre les extrémités des conduites, lequel tube de communication (26) est fermé par une partie de tête (26b) qui peut être retirée du tube de communication par une pression exercée par les doigts sur l'extérieur du tube de connexion (28).

3. Agencement de traitement des liquides du corps selon la revendication 1, caractérisé en ce que le moyen à fermeture interne (21) comprend un élément de communication circulaire (29) dont le diamètre est plus important que le diamètre interne du tube de connexion (28) pour fermer le passage passant par le tube de connexion (28) et qui peut être basculé par une pression exercée par les doigts sur l'extérieur du tube de connexion (28).

FIG_1

FIG_2

FIG_3

FIG_4